# EUROPÄISCHE PATENTANMELDUNG

(11) **EP 1 367 171 A1**
(43) Veröffentlichungstag der Anmeldung: **03.12.2003**
(21) Anmeldenummer: 02012039.0
(22) Anmeldetag: 31.05.2002
(51) Int. Cl.: D06H 3/08, G01N 33/34, G01N 33/36, G01N 21/89

(54) **Sensorleiste mit Sensoren**

(71) Anmelder: Hergeth, Hubert A., 4731 Eynatten (BE)
(72) Erfinder: Hergeth, Hubert A., 4731 Eynatten (BE)

(57) **Zusammenfassung**

Ein Sensor zur Erfassung von Materialbahnen wird auf mehreren Platinen (1) mit Fotoelement zusammengesezt. Der abstand der Platinen (1) wird durch mechanische Elemente (4) festgelegt.

## Beschreibung

Die Erfindung betrifft einen Sensor zum Kontrollieren von Materialbahnen z. B. in der Textilindustrie. Der Sensor besteht aus einem Gehäuse, das sich mindestens über die Breite der Materialbahn erstreckt. In dem Gehäuse ist eine Beleuchtung und eine Reihe von Sensoren angebracht, die das Licht von der Faserbahn erfassen. Um die Materialbahn gut zu erkennen, sind eine hohe Dichte von Sensoren z. B. mehr als fünf Sensoren pro mm Bahnbreite notwendig. Es ist deshalb notwendig, solche Sensoren, die sich zu mehreren auf Chips befinden, auf eine Platine zu bonden. Es ist aber problematisch, Platinen von großer Länge, z. B. 3 m, wie sie für textile Faserbahnen benötigt werden, herzustellen und mit Chips zu bestücken. Die Erfindung löst diese Aufgabe auf einfache Weise. In dem Gehäuse, vorzugsweise aus extrudiertem Aluminium, sind mindestens zwei Führungsnuten vorhanden, in die mehrere Platinenstreifen nacheinander eingeführt werden. Die Platinen sind mit mindestens einer Reihe Chips bestückt, die mehrere Fotosensoren in Reihe beinhalten. Bei diesen Chips befinden sich die Fotosensoren bis an den Rand des Chips. Bei normalen Zeilensensoren z. B. reichen die Fotosensoren nicht bis an den Rand des Chips, sondern sind von einem Rahmen eingefaßt.

Auf der Platine sind die Fotosensoren so angeordnet, daß der erste oder letzte Fotosensor des Fotochips mit der Platine abschließt oder über die Platine hinausragt. Um zu verhindern, daß die Chips beim Aneinanderreiben der Platinen beschädigt werden, sind an den Enden der Platinen mechanische Abstandshalter angebracht. Diese sind vorzugsweise Schraubenköpfe von Schrauben, die in Bohrungen der Platine stecken. Da die Bohrungen in einer Platine zu den Befestigungsbohrungen der Chips relativ sehr genau sind, können so kleinste Distanzen zwischen den Anfangs- und Endchips eingehalten werden, ohne goßen Aufwand zu treiben. Durch das so ermöglichte Aneinanderreihen von Platinen mit Chips sind sehr lange Bahnsensoren für große Materialbahnen einfach möglich.

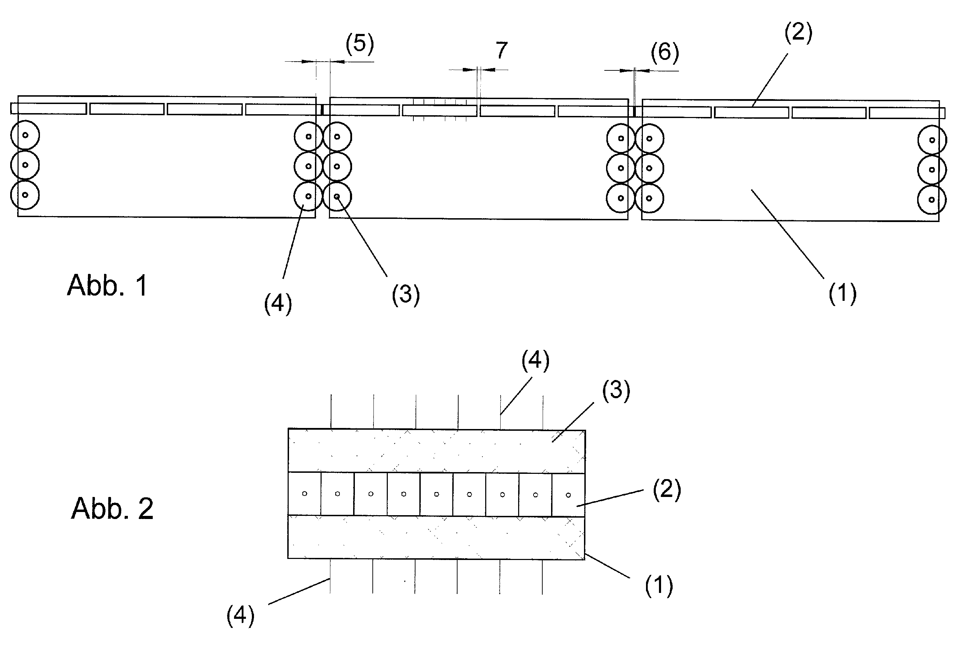

Abbildung 1 zeigt drei Platinen, wie sie aneinander gereiht sind. Die Platinen (1) sind mit Chips (2) bestückt. Der Abstand der Platinen zueinander wird durch Schraubenköpfe (4) eingehalten. Die Schraubenschäfte sind in Bohrungen (3) in der Platine positioniert. Auf dieser Weise wird eine Distanz (5) zwischen den Platinen eingehalten. Die Chips (2) sind auf der Platine mit einem Abstand (7) zueinander gebondet. Dieser Abstand (7) entspricht in etwa dem Abstand (6) der Chips zueinander, die an den Rändern der Platine plaziert sind.

Abbildung 2 zeigt einen Chip (1) mit lichtempfindlichen Elementen (2). Die lichtempfindlichen Elemente reichen bis an den Rand des Chips heran. Gebräuchliche Chips für Zeilenkameras haben einen breiten Rand (3) ganz um die Sensorelemente herum. Die elektrischen Kontakte (4) sind an den Längsseiten des Chips angebracht.

## Patentansprüche

1. Sensor zum Kontrollieren von Materialbahnen bestehend aus einem Gehäuse, in dem sich eine Beleuchtung, eine Optik und eine Reihe von Sensoren befinden, daduch gekennzeichnet, daß in dem Gehäuse mehrere Platinen, die auf dem Chips mit fotosensitiven Sensoren angebracht sind, aneinandergereiht sind und der Abstand zwischen den Sensoren der Chips auf einer Platine etwa gleich dem Abstand der Sensoren der Chips an den Rändern der Platine ist.

2. Vorrichtung nach Anspruch 1) **dadurch gekennzeichnet, daß** der Abstand der Sensoren der Chips auf der Platine und der Abstand der Sensoren bei benachbarten Platinen um maximal 60 % variiert.

3. Vorrichtung nach Anspruch 1) und 2) **dadurch gekennzeichnet, daß** der Abstand nach Anspruch 2) maximal 1 mm beträgt.

4. Vorrichtung nach einem der Ansprüche 1) bis 3) **dadurch gekennzeichnet, daß** der Abstand der Platinen durch Abstandhalter, die auf den Platinen angebracht sind, eingehalten wird.

5. Vorrichtung nach einem der Ansprüche 1) bis 4) **dadurch gekennzeichnet, daß** die Abstandhalter in Löchern auf den Platinen befestigt sind.

6. Vorrichtung nach einem der Ansprüche 1) bis 5) **dadurch gekennzeichnet, daß** die Abstandhalter die Köpfe von schraubenartigen Befestigungseelementen sind.

7. Vorrichtung nach einem der Ansprüche 1) bis 6) **dadurch gekennzeichnet, daß** der Abstand der Platinen zueinander mittels Abstandshalter einstellbar ist.

8. Vorrichtung nach einem der Ansprüche 1) bis 7) **dadurch gekennzeichnet, daß** die Platinen in Nuten des Sensorgehäuses geführt werden können.
